# EUROPÄISCHE PATENTSCHRIFT

(11) **EP 2 890 335 B1**
(45) Veröffentlichungstag und Bekanntmachung des Hinweises auf die Patenterteilung: **11.10.2017**
(21) Anmeldenummer: 13758745.7
(22) Anmeldetag: 29.08.2013
(51) Int. Cl.: A61F 2/78, A61F 2/80, A61F 2/74

(54) **PROTHESENLINER UND PROTHESENSCHAFTSYSTEM MIT PROTHESENLINER UND PROTHESENSCHAFT**
PROSTHESIS LINER, AND PROSTHESIS SOCKET SYSTEM WITH PROSTHESIS LINER AND PROSTHESIS SOCKET
MANCHON PROTHÉTIQUE ET SYSTÈME D'EMBOÎTURE DE PROTHÈSE COMPRENANT UN MANCHON ET UNE EMBOÎTURE

(30) Priorität: 31.08.2012 DE 102012017214
(43) Veröffentlichungstag der Anmeldung: 08.07.2015
(73) Patentinhaber: Otto Bock HealthCare GmbH, 37115 Duderstadt (DE)
(72) Erfinder: MÜLLER, André, 37115 Duderstadt (DE); HILLMANN, Martin, 37115 Duderstadt (DE)
(74) Vertreter: Stornebel, Kai
(86) Internationale Anmeldenummer: PCT/EP2013/002595
(87) Internationale Veröffentlichungsnummer: WO 2014/032802

(56) Entgegenhaltungen:
- DE-A1- 10 142 491
- DE-A1-102004 050 201
- US-A1- 2010 070 051
- US-A1- 2012 191 217
- US-A1- 2012 191 218
- US-B1- 6 797 008

## Beschreibung

Die Erfindung betrifft einen Prothesenliner zur Anlage an einen Stumpf, mit einem elastischen Grundkörper, der eine proximale Öffnung zum Einführen des Stumpfes in einen Aufnahmeraum und ein distales Ende aufweist, sowie ein Prothesenschaftsystem mit einem solchen Prothesenliner und einem Prothesenschaft.

Prothesen dienen zum Ersetzen der Funktion und gegebenenfalls der optischen Erscheinung einer fehlenden Gliedmaße und werden an dem Körper des Patienten festgelegt. Zur Festlegung der Prothese sind vielfältige Möglichkeiten bekannt. Eine weit verbreitete Möglichkeit zur Festlegung von Prothesen an den Extremitäten ist die Anordnung an einem verbliebenen Teil der Gliedmaße, dem sogenannten Stumpf. Der Stumpf wird von einem Prothesenschaft umfasst, der in der Regel formstabil ist. An dem proximalen Ende des Stumpfes ist eine Einführöffnung ausgebildet, an dem distalen Ende des Schaftes ist zumindest eine Befestigungseinrichtung vorgesehen, an der weitere Prothesenkomponenten angeordnet werden können, beispielsweise Gelenke oder funktionale Einheiten wie Prothesenfüße oder Prothesenhände. Um eine gute Passform zu erreichen, wird ein Modell des Stumpfes angefertigt und der Schaft an die Kontur des Stumpfmodells angepasst. Um Volumenschwankungen ausgleichen zu können, kann vorgesehen sein, dass der Schaft enger gefertigt wird als das abgeformte Stumpfmodell.

Um den Tragekomfort zu erhöhen, kann es vorgesehen sein, dass zwischen dem Prothesenschaft und dem Stumpf ein sogenannter Prothesenliner angeordnet ist. Der Prothesenliner besteht in der Regel aus einem geschlossenen Grundkörper mit einer proximalen Öffnung und wird strumpfartig über den Stumpf gezogen. Das elastische Material haftet auf der Stumpfoberfläche und stellt die Verbindung zwischen dem Stumpf und dem Prothesenschaft her. Zur Befestigung des Prothesenliners an dem Prothesenschaft können mechanische Verriegelungselemente am distalen Ende des Prothesenliners und korrespondierende Verriegelungseinrichtungen am distalen Ende des Prothesenschaftes vorgesehen sein, die den Prothesenliner mit dem Prothesenschaft nach dem Einsteigen in den Prothesenschaft formschlüssig verriegeln. Über eine Entriegelungseinrichtung kann die Befestigung des Prothesenliners an dem Prothesenschaft aufgehoben werden.

Eine weitere Möglichkeit zur Befestigung eines Prothesenschaftes an einem Stumpf besteht in der sogenannten Saugschaftlinertechnologie, bei der der Prothesenschaft luftdicht gegenüber dem Prothesenliner abgedichtet wird und aus dem Zwischenraum zwischen dem Prothesenliner und dem Prothesenschaft die darin vorhandene Luft abgesaugt oder herausgedrückt wird. Ein Rückströmen wird über ein Rückschlagventil verhindert. Hierzu ist es notwendig, den Prothesenschaft luftdicht auszugestalten und eine großflächige Abdichtung gegenüber dem Prothesenliner sicherzustellen.

Die DE 101 53 796 A1 betrifft einen Prothesenschaft und einen Prothesenliner, der an seinem distalen Ende ein Fixierglied mit einem zylindrischen Zapfen aufweist, der mit einem rotationssymmetrischen Sägezahnprofil versehen ist. Der Zapfen wird durch eine Öffnung am distalen Ende des Prothesenschaftes hindurchgeführt und über einen Schieber mechanisch verriegelt. An dem distalen Ende des Prothesenliners ist um den Zapfen herum eine Dichtlippe als geschlossener Ring angeordnet.

Die US 2011/0035027 A1 betrifft ein vakuumunterstütztes Linersystem mit einem flexiblen Liner, der über einen Stumpf gezogen wird und zumindest eine umlaufende Kante aufweist. Der Liner ist aus einem luftundurchlässigen Material gefertigt und hat zumindest einen porösen Bereich, der von der umlaufenden Kante beabstandet ist, um den Transport von Luft und Feuchtigkeit zwischen einer äußeren Oberfläche und einer inneren Oberfläche des Liners zu ermöglichen.

Die DE 101 42 491 A1 betrifft eine Dichtanordnung zum Abdichten eines Amputationsstumpfes in einem becherförmigen, luftdichten Prothesenschaft mit einem auf den Amputationsstumpf aufziehbaren, schlauchförmigen, elastischen und luftdichten Träger, der den Amputationsstumpf in Umfangsrichtung allseitig umschließt. An der Außenseite des Trägers ist eine Dichtlippe angeordnet, die an dem Prothesenschaft abdichtend anliegt.

Die US 8,052,760 B2 betrifft einen Prothesenliner zur Ausbildung eines Interfaces zwischen einem Stumpf und einem Prothesenschaft. Der Liner ist im Wesentlichen konisch ausgebildet und aus einem Material hergestellt, das elastisch verformbar ist. Das proximale Ende ist offen, das distale Ende ist geschlossen. An dem äußeren Umfang des Liners sind separate Dichtelemente an der äußeren Oberfläche des Liners befestigt.

Die WO 2012/051385 A1 betrifft einen Prothesenliner als Interface zwischen einem Stumpf und einem Prothesenschaft, bei dem zumindest ein federndes Dichtungselement sich radial nach außen und zumindest über einen Teil des Umfanges des Liners erstreckt. Ein Kissen zur Steuerung des Volumens ist in dem Liner angeordnet und drückt das Dichtungselement nach außen.

Die US 2012/0191217 A1, die den nächstliegenden Stand der Technik darstellt, betrifft ein Schaftsystem mit einem Vakuumliner für prothetische oder orthetische Vorrichtungen. An dem geschlossenen Liner ist an dem distalen Ende ein Bereich aus einem elastischen Material angeordnet, das härter als das Material des übrigen Liners ist. Der Bereich weist einen konkaven Abschnitt auf, der sich von einer äußeren Endoberfläche des Bereiches nach innen erstreckt. Der distale Bereich und der konkave Abschnitt bilden zumindest einen Teil einer Vakuumpumpe aus, um Luft aus dem Zwischenraum zwischen dem Liner und dem Prothesenschaft herauszupumpen.

Die US 2012/191218 A1 betrifft eine Stützvorrichtung für eine Prothese oder Orthese mit einem Prothesenschaft und einem darin angeordneten Prothesenliner. An dem distalen Ende des Prothesenliners ist eine federnde Schicht in Gestalt eines Kissens angeordnet. Zwischen dem Schaft und dem Kissen kann eine poröse Verteilungsschicht angeordnet sein. In der Außenseite des Kissens sind kegelförmige Ausnehmungen eingeformt.

Die US 2010/0070051 A1 betrifft einen becherförmigen Prothesenschaft aus luftdichtem und im Wesentlichen steifem Material mit Abdichtungsmitteln gegen einen Lufteinritt von der proximalen, offenen Seite. In einem Hohlraum in einem Liner oder zwischen einem Liner und einem Prothesenschaft ist eine Pumpblase angeordnet, über die Luft aus dem Zwischenraum zwischen der Außenseite des Liners und der Innenseite des Schaftes herausgepumpt werden kann. Zwischen der Innenseite des Liners und der Innenseite des Schaftes kann ein Verbindungskanal in dem Prothesenliner ausgebildet sein.

Die US 6 797 008 B1 betrifft ein System und ein Verfahren zur Sicherung einer Prothese an einem Stumpf mit einem Schaft und einem in dem Schaft angeordneten Saugnapf, der an einer Seitenwand des Schaftes oder an dessen distalem Ende angeordnet ist. Zusätzlich ist eine mechanische Sicherung über einen Seilzug oder einen Verriegelungsstift vorgesehen, wenn ein Liner an einem Stumpf angeordnet wird.

Aufgabe der vorliegenden Erfindung ist es, einen Prothesenliner und einen Prothesenschaft bereitzustellen, mit dem eine Befestigung der Prothese auch bei verminderter Passgenauigkeit im Schaft gewährleistet ist und Volumenschwankungen weniger Einfluss auf die Festlegung innerhalb des Prothesenschaftes haben.

Erfindungsgemäß wird diese Aufgabe durch einen Prothesenliner mit den Merkmalen des Hauptanspruche und ein Prothesenschaftsystem mit den Merkmalen des nebengeordneten Anspruchs gelöst. Vorteilhafte Ausgestaltungen und Weiterbildungen der Erfindung sind in den Unteransprüchen, den Figuren und der Beschreibung offenbart.

Der Prothesenliner zur Anlage an einen Stumpf, mit einem elastischen Grundkörper, der eine Proximalöffnung zum Einführen des Stumpfes in einen Aufnahmeraum und distales Ende aufweist, sieht vor, dass an der Außenseite des Prothesenliners ein Saugnapf angeordnet ist. Der Saugnapf oder die Saugnäpfe ist oder sind als ein separates Bauteil ausgebildet und an dem Prothesenliner befestigt. Die Festlegung kann über die beschriebenen Befestigungseinrichtungen erfolgen. Grundsätzlich ist es auch möglich aber nicht von der Erfindung umfasst, dass der Saugnapf an dem distalen Ende des Prothesenliners oder der Abschlusskappe angeformt ist, so dass sich eine sehr kompakte Bauweise ergibt. Darüber hinaus ist aufgrund der elastischen Verformbarkeit des Saugnapfes eine gute und elastische Abstützung des distalen Stumpfendes innerhalb des Prothesenliners gegeben. Auch bei einer einstückigen Ausgestaltung des Prothesenliners mit dem Saugnapf ist die funktionale Trennung zwischen der Gewährleistung einer ausreichenden Haltekraft über den Saugnapf am distalen Ende von der Positionierung und Sicherung des Prothesenliners innerhalb des Prothesenschaftes vorhanden. Der Prothesenliner ist dazu eingerichtet, an einem Prothesenschaft über einen Unterdruck zwischen dem Saugnapf und dem Prothesenschaft festgelegt zu werden, wobei zwischen dem Prothesenliner und dem Prothesenschaft kein Unterdruck anliegt. Statt einer ganzflächigen Vakuumlösung, bei der das Volumen zwischen dem Prothesenschaft und dem Prothesenliner evakuiert wird, ist erfindungsgemäß die Festlegung des Prothesenliners innerhalb des Prothesenschaftes nur am distalen Ende über den Saugnapf vorgesehen.

Um eine sichere Befestigung des Saugnapfes an dem Prothesenliner gewährleisten zu können und darüber hinaus eine ausreichende Stabilität und Kraftverteilung von dem distalen Ende des Prothesenliners auf den Stumpf zu ermöglichen, ist in einer Weiterbildung der Erfindung am distalen Ende eine formstabile Abschlusskappe vorgesehen, an der der Saugnapf festgelegt wird.

Der Saugnapf ermöglicht es, die Haltekraft zwischen dem Prothesenliner und dem Prothesenschaft mit Hilfe eines Zusatzelementes herzustellen. Dazu wird Unterdruck im Bereich zwischen dem Saugnapf und dem Schaft erzeugt. An dem Liner selbst liegt kein Unterdruck an, präziser gesagt liegt in dem Volumen zwischen dem Prothesenschaft und dem Prothesenliner kein Unterdruck an, so dass die Passgenauigkeit eines Prothesenschaftes nicht mehr entscheidend für die Dichtigkeit der Verbindung zwischen dem Prothesenschaft und dem Prothesenliner und damit für die Haltekraft des Prothesenliners innerhalb des Prothesenschaftes ist. Der Saugnapf kann an dem distalen Ende des Liners angeordnet sein. Durch die Anordnung eines Saugnapfes an dem distalen Ende des Prothesenliners kann jeder beliebige Liner zur Befestigung innerhalb eines Prothesenschaftes verwendet werden, besondere Anforderungen an die Dichtigkeit und insbesondere die Abdichtfähigkeit zum Prothesenschaft werden bis auf den Bereich der Anlage im Prothesenschaft nicht gestellt. Es ist auch möglich und vorgesehen, dass neben der Anordnung des Saugnapfes an dem vorderen, also distalen Ende des Liners eine Anordnung eines oder mehrerer Saugnäpfe im vorderen Endbereich erfolgt, so dass eine Verteilung der Haltekräfte auf mehrere Stellen bewirkt wird. Die mehreren Saugnäpfe können verteilt über den Umfang an der Außenseite angeordnet sein, beispielsweise in einer symmetrischen Anordnung, ebenso kann ein zentraler Saugnapf von weiteren am Umfang angeordneten Saugnäpfen ergänzt werden.

Der Prothesenschaft selbst kann weitgehend perforiert ausgebildet sein, so dass eine Ventilation stattfinden kann, ebenso können Flüssigkeiten in das Volumen zwischen dem Prothesenliner und dem Prothesenschaft eindringen und durch Öffnungen wieder austreten, ohne dass die Gefahr besteht, dass sich der Prothesenliner von dem Prothesenschaft löst. Über eine Definition der Saugnapfgeometrie ist es möglich, die Haltekraft exakt einzustellen, ohne dass die geometrischen Bedingungen am Prothesenschaft oder Prothesenliner berücksichtigt werden müssten.

Insbesondere kann der jeweilige Saugnapf reversibel an dem distalen Ende oder im distalen Endbereich des Prothesenliners befestigt sein. Durch eine reversible Befestigung oder Befestigungsmöglichkeit an dem Prothesenliner ist es möglich, unterschiedlich große Saugnäpfe an dem Liner zu befestigen, um eine Anpassung an die jeweils benötigte Haltekraft ermöglichen zu können. Ebenso ist es möglich, dass an einen Prothesenschaft angepasste Saugnäpfe an unterschiedliche Prothesenliner montiert werden können oder Standardsaugnäpfe an individuell gefertigte Prothesenliner und Prothesenschäfte anzubringen. Zur reversiblen Befestigung des Saugnapfes an dem Prothesenliner ist zumindest eine Befestigungseinrichtung an dem Prothesenliner und korrespondierend an dem Saugnapf ausgebildet. Die Befestigungseinrichtung kann beispielsweise ein Schraubsystem, ein Klicksystem mit einer Kugelkopflagerung, eine formschlüssige Verriegelung über Klettverschlüsse oder ein Zapfensystem mit korrespondierender Aufnahme und formschlüssiger Verriegelung ausgebildet sein.

Der Saugnapf weist zumindest eine umlaufende Dichtlippe auf, über die das Volumen zwischen dem Saugnapf und dem Prothesenschaft abdichtbar ist. Die umlaufende Dichtlippe definiert die Dichtkante, die sich dauerhaft an der Innenseite des Prothesenschaftes oder einer Aufnahmeeinrichtung anlegt, um den notwendigen Unterdruck aufrecht zu erhalten. Wird der Saugnapf, der aus einem elastischen Material ausgeführt ist, beispielsweise einem TPE, in den Prothesenschaft eingeführt, führt das Herausdrücken des Gases, in der Regel Luft, aus dem eingeschlossenen Volumen zu einem Unterdruck. Je nach Ausgestaltung sowohl des Saugnapfes als auch des Prothesenschaftes oder einer Aufnahmeschale, kann ein Teil des Saugnapfes umklappen und gegen die Innenseite des Prothesenschaftes oder der Aufnahmeeinrichtung drücken, wodurch eine Dichtkante gegenüber dem Schaft ausgebildet wird. Es besteht die Möglichkeit, dass mehrere Dichtkanten an der Unterseite des Saugnapfes ausgebildet sind, beispielsweise durch das Anbringen oder Ausbilden einer Lamellenstruktur auf der Unterseite des Saugnapfes, die aus konzentrisch angeordneten Lamellen besteht. Dadurch kann eine Anpassung an unterschiedliche Durchmesser oder Konturen innerhalb des Prothesenschaftes erreicht werden. Ebenfalls wird bei mehrfacher Ausgestaltung von Dichtkanten oder Dichtlippen die Sicherheit dadurch erhöht, dass mindestens immer eine Dichtlippe im radial dichtenden Kontakt mit der Innenseite des Prothesenschaftes steht.

Innerhalb der umlaufenden Dichtlippe oder der umlaufenden Dichtlippen kann an der Saugnapfoberfläche eine aufgeraute Struktur, ein Textil und/oder eine nicht haftende oder luftdurchlässige Beschichtung vorgesehen sein, wodurch sichergestellt wird, dass nur die gewünschte Dichtlippe eine Dichtkante ausgebildet und in einem abdichtenden Kontakt mit der Innenseite des Prothesenschaftes tritt. Dadurch wird weiterhin gewährleistet, dass die Wirkfläche des Vakuums über die gesamte Unterseite des Saugnapfes innerhalb der Dichtlippe flächig verteilt wird. Es wird verhindert, dass an der Unterseite versehentlich an einer anderen Stelle ein Dichtkontakt mit dem Schaft entsteht, was zur Folge hätte, dass die Haltekraft kleiner als gewünscht ausfällt.

Vorteilhafterweise weist der Saugnapf eine auf der dem Prothesenliner abgewandten Seite eine konvexe Form auf, die dazu führt, dass sich der Saugnapf beim Einführen in den Prothesenschaft und dem Herausdrücken des Volumens unterhalb der konvexen Wölbung an die Innenseite des Prothesenschaftes anlegt. Dazu ist es notwendig, dass der äußere Rand umklappt, wodurch eine radial nach außen wirkende Kraft auf den äußeren Rand des Saugnapfes aufgrund der elastischen Rückstellkraft ausgeübt wird. Durch das Umklappen wird eine zusätzliche mechanische Verriegelung bereitgestellt, da gegen das Herausziehen des Prothesenliners aus dem Prothesenschaft zusätzlich zu dem Unterdruck auch noch die mechanische Rückstellkraft überwunden werden muss. Der Saugnapf weist vorteilhafterweise eine Form und Funktionalität auf, die ein vollumfängliches Umklappen zumindest eines Randbereiches des Saugnapfes ermöglicht, wobei vorteilhafterweise eine Rückstellkraft durch den Saugnapf aufgebracht wird, die den umgeklappten Teil des Saugnapfes wieder in die Ausgangsstellung drängt.

Eine Weiterbildung der Erfindung sieht vor, dass eine strömungstechnische Verbindung zwischen dem Aufnahmeraum innerhalb des Prothesenliners und dem Saugnapf besteht. Dadurch ist es möglich, durch wiederholtes Belasten und Entlasten des Saugnapfes innerhalb des Prothesenschaftes eine Pumpwirkung zwischen dem Aufnahmeraum des Prothesenliners und dem Stumpf zu bewirken. Dadurch ist es möglich, eine Evakuierung des Zwischenraumes zwischen dem Prothesenliner und dem Stumpf vorzunehmen. Darüber hinaus ist es möglich, einen Luftaustausch durch eine geregelte Leckage innerhalb des Liners vorzunehmen. Dies führt zu einer erhöhten Hautverträglichkeit, da Luft und Feuchtigkeit aus dem Inneren des Prothesenliners abtransportiert werden können.

Innerhalb der strömungstechnischen Verbindung zwischen dem Aufnahmeraum für den Stumpf und dem Saugnapf kann eine Sperrvorrichtung angeordnet sein, die ein Rückströmen von Luft oder Feuchtigkeit in den Aufnahmeraum verhindert. Dadurch kann gezielt ein Vakuum oder Unterdruck innerhalb des Prothesenliners erreicht werden. Der Saugnapf dient weiterhin als mechanische Halterung zwischen dem Prothesenschaft und dem Prothesenliner, gleichzeitig wird ein sicherer Sitz des Prothesenliners an dem Stumpf durch ein gezieltes Evakuieren möglich. Durch die Sperreinrichtung, beispielsweise ein Rückschlagventil, kann der Grad des Evakuierens eingestellt werden.

An dem proximalen Ende des Prothesenliners kann ein zu dem Stumpf hin dichtender Abschnitt ausgebildet sein, um eine luftdichte und wasserdichte Anlage zumindest des proximalen Randes des Liners an dem Stumpf zu erreichen. An diesen oberen, dichtenden Abschnitt kann sich distal dazu ein Bereich anschließen oder kann dazu ein Bereich angeordnet sein, der zurückgesetzt ausgebildet ist, so dass sich ein beabstandeter Bereich bzw. Freiraum zwischen dem Stumpf und dem Liner ausbildet. Innerhalb dieses Bereiches können eine raue Struktur, Kanäle, ein Textil, ein Schaumstoff oder eine für luftdurchlässige Beschichtung vorgesehen sein, so dass innerhalb dieses Bereiches eine Luftzirkulation stattfinden kann. Ebenfalls ist es möglich, durch gezieltes Evakuieren dieses Bereiches beispielsweise durch die strömungstechnische Verbindung zwischen dem Aufnahmeraum und dem Saugraum eine gezielte Leckage und einen Luftaustausch zu realisieren.

Das Prothesenschaftsystem mit einem Prothesenliner, wie er oben beschrieben wurde und einem Prothesenschaft sieht vor, dass der Prothesenschaft einen zu dem Saugnapf korrespondieren Anlagebereich aufweist und zwischen dem Saugnapf und dem Anlagebereich im gekoppelten Zustand ein geschlossenes Volumen ausgebildet ist. Der korrespondierende Anlagebereich ist so ausgebildet, dass sich der Saugnapf an der Innenseite des Prothesenliners entlang einer Dichtlippe dichtend anlegen kann, um eine Dichtkante auszubilden, so dass das Umgebungsmedium, in der Regel Luft, nicht in das geschlossene Volumen eindringen kann. Das geschlossene Volumen weist im Verhältnis zum Umgebungsdruck einen Unterdruck auf, so dass eine Haltekraft über den Saugnapf aufgebracht wird, so dass der Prothesenliner nicht aus dem Prothesenschaft entfernt werden kann. Der Unterdruck oder das Vakuum in dem Anlagebereich kann durch das Einführen des Saugnapfes in den Prothesenschaft und ein mechanisches Herausdrücken zumindest eines Teils der eingeschlossenen Luft erreicht werden, alternativ oder ergänzend kann eine Pumpeinrichtung vorgesehen sein, über die Luft aus dem Volumen zwischen dem Saugnapf und dem Prothesenschaft abgesaugt wird.

Neben einem seitlichen Vorbeidrücken der Luft an der Umfangskante des Saugnapfes ist es möglich, dass innerhalb des Volumens eine Sperreinrichtung oder eine strömungstechnische Verbindung zu einer Sperreinrichtung vorhanden ist, die ein Ausströmen von Luft ermöglicht und ein Rückströmen verhindert. Dies ist beispielsweise durch ein Rückschlagventil möglich, das gegebenenfalls auch geöffnet werden kann, um die Verriegelung aufzuheben. Durch das Anordnen einer Sperreinrichtung ist ein nahezu geräuschloses Evakuieren möglich, darüber hinaus kann derjenige Ort definiert werden, an dem die Luft aus dem Volumen herausgedrückt wird.

Das geschlossene Volumen kann mit einer Belüftungseinrichtung zum Einleiten von Luft in strömungstechnischer Verbindung stehen, um den Unterdruck in dem geschlossenen Volumen aufzuheben und den Prothesenliner aus dem Prothesenschaft entfernen zu können. Die Belüftungseinrichtung weist einen Zugang zu dem geschlossenen Volumen unterhalb der Dichtkante auf, um ein Lösen der Verbindung zu ermöglichen.

Eine Evakuierungspumpe kann mit dem geschlossenen Volumen in strömungstechnischer Verbindung stehen, um eine initiale Evakuierung oder eine Aufrechterhaltung des Unterdrucks auch in einem statischen Zustand zu gewährleisten. Die Evakuierungspumpe kann in einem Druckbetrieb betreibbar sein, um das geschlossene Volumen zu befüllen, die Pumpe im Druckbetrieb dient dann als Belüftungseinrichtung zum Einleiten von Luft.

An dem distalen Ende des Prothesenschaftes kann eine Aufnahmeschale mit konkaver Wölbung angeordnet sein. Durch die konvexe Wölbung des Saugnapfes, also eine Wölbung, die von dem Liner weg gerichtet ist, und eine entgegensetzt gewölbte Aufnahmeschale, die als separates Bauteil gefertigt oder integraler Bestandteil des Prothesenschaftes sein kann, ergibt sich eine Deckelfunktion des Saugnapfes vor dem Einführen bzw. vor der Verformung des Saugnapfes an dem Anlagebereich bzw. der Aufnahmeschale. Bei einer konkaven Wölbung der Aufnahmeschale bzw. des Anlagebereiches ist es möglich, dass ein sehr großer Saugnapfdurchmesser verwendet wird, da sich der elastische Saugnapf dann verformt und eine entgegengesetzte Wölbung, nämlich eine konkave Wölbung einnimmt. Aufgrund der dadurch möglichen großen Saugnapffläche ergibt sich eine korrespondierend große Haltekraft gegenüber einer relativ kleinen Saugnapfoberfläche bei gleichbleibendem Unterdruck. Die Aufnahmeschale kann einen nach innen vorstehenden Ring oder Vorsprung aufweisen, um eine zusätzliche mechanische Verriegelung des umgeklappten Saugnapfes entgegen das Herausziehen aus dem Anlagebereich, der Aufnahmeschale oder dem Prothesenschaft zu wirken.

Zwischen dem Aufnahmeraum innerhalb des Prothesenliners und dem geschlossenen Volumen zwischen dem Saugnapf und dem Prothesenschaft im Anlagebereich kann eine strömungstechnische Verbindung bestehen, die eine Sperreinrichtung aufweisen kann, die ein Rückströmen in den Aufnahmeraum verhindert. Hierdurch ist es möglich und vorgesehen, dass eine Evakuierungspumpe dem Prothesenschaft zugeordnet und insbesondere daran angeordnet ist, um das Volumen zwischen dem Prothesenschaft und dem Saugnapf zu evakuieren.

Im Anlagebereich des Prothesenschaftes kann eine Oberfläche mit aufgerauter Struktur, einem Textil und/oder einer nichthaftenden bzw. luftdurchlässigen Beschichtung vorgesehen sein, die korrespondierend zu der Oberflächenbeschaffenheit innerhalb der Dichtlippe ausgebildet ist. Diese Struktur oder Beschichtung in dem Anlagebereich ist von einem Bereich umgeben, in dem die Dichtlippe dichtend anliegen kann; ggf. wird die Luftdurchlässigkeit und damit die gezielte Nichthaftung durch ein Zusammenwirken der Beschichtungen oder Strukturen an dem Saugnapf und dem Anlagebereich ausgebildet.

Nachfolgend werden Ausführungsbeispiele der Erfindung anhand der beigefügten Figuren näher erläutert. Gleiche Bezugszeichen bezeichnen gleiche Komponenten. Es zeigen:
- Figur 1: eine schematische Darstellung eines Prothesenliners und eines Saugnapfes in Seitenansicht;
- Figur 2: eine perspektivische Darstellung gemäß Figur 1;
- Figur 3: eine Seitenansicht eines Prothesenschaftes und eines Prothesenliners vor dem Fügen;
- Figur 4: eine Schnittansicht durch einen Prothesenliner während des Fügens;
- Figur 5: eine Schnittansicht im gefügten Zustand;
- Figur 6: eine Schnittdarstellung eines Saugnapfes;
- Figur 7: eine perspektivische Darstellung eines Prothesenliners mit Saugnapf vor dem Fügen mit einer Aufnahmeschale;
- Figur 8: eine Schnittansicht der Figur 7;
- Figur 9: eine Darstellung gemäß Figur 8 während des Fügens;
- Figur 10: eine Darstellung gemäß Figur 8 im gefügten Zustand;
- Figur 11: eine Schnittdarstellung eines Prothesenschaftsystems mit Pumpe;
- Figur 12: eine Variante der Erfindung in Schnittansicht;
- Figur 13: eine perspektivische Ansicht einer Aufnahmeschale von schräg unten;
- Figur 14: eine Aufnahmeschale in perspektivischer Darstellung von schräg oben;
- Figur 15: eine Einzeldarstellung eines Prothesenliners mit Saugnapf im gefügten Zustand sowie
- Figur 16: eine Darstellung gemäß Figur 15 während des Entnehmens des Saugnapfes.

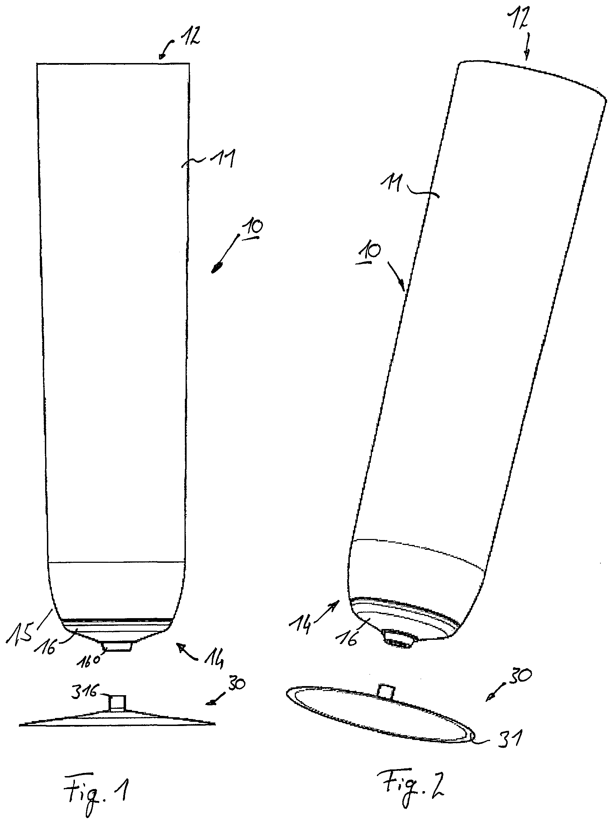

Figur 1 zeigt einen Prothesenliner 10 mit einem elastischen Grundkörper 11, der beispielsweise aus Silikon oder einem anderen Elastomer hergestellt sein kann. An dem proximalen Ende 12 des Prothesenliners ist eine Öffnung vorhanden, durch die ein Prothesennutzer in einen Aufnahmeraum innerhalb des Prothesenliners 10 einsteigen kann. Der Aufnahmeraum ist in dieser Figur nicht gezeigt. Das Anlegen des Prothesenliners erfolgt in der Regel durch Herabrollen des proximalen Endes 12 oder Umstülpen des proximalen Endes 12, Anlegen eines distalen Endes 14 des Prothesenliners an das distale Ende des Stumpfes und anschließendes Abrollen auf den Stumpf. An dem distalen Ende 14 des Prothesenliners 10 ist im dargestellten Beispiel eine formstabile Abschlusskappe 16 angeordnet, die zur stabilen und sanften Bettung des Stumpfes gegenüber dem Anschluss eines Saugnapfes 30 auf der Außenseite 15, also der dem Aufnahmeraum gegenüberliegenden Seite des Prothesenliners 10, dient. An der Abschlusskappe 16 sind Befestigungseinrichtungen 160 angeordnet, über die es möglich ist, den Saugnapf 30, der mit einer in distale Richtung ausgebildeten Wölbung von dem Liner 10 weg versehen ist, an der Abschlusskappe 16 anzuordnen. Der Saugnapf 30 verfügt ebenfalls über eine Befestigungseinrichtung 316, über die der Saugnapf 30 an dem Prothesenliner 10, insbesondere an der distalen Abschlusskappe 16 festlegbar ist.

In der Figur 2 ist die Unterseite des Saugnapfes 30 zu erkennen, ebenso ist die Dichtlippe 31 am äußeren Umfang des Saugnapfes 30 zu erkennen. Durch die Anordnung der Dichtlippe 31 am äußeren Umfang des Saugnapfes 30 wird eine maximale Fläche bereitgestellt, so dass auch bei einem relativ geringen Unterdruck eine hohe Haltekraft realisiert werden kann. Der Saugnapf 30 ist über die Befestigungselemente 160, 316 reversibel an dem Prothesenliner 10 befestigbar. Dadurch ist es möglich, unterschiedliche Saugnapftypen an dem Liner festzulegen. Die Saugnäpfe 30 können sich hinsichtlich Durchmesser, Formgebung oder Materialwahl unterscheiden.

Die Kopplung des Saugnapfes 30 mit dem Prothesenliner 10 ist vorzugsweise als eine mechanische Kopplung, beispielsweise als Schraubkopplung ausgeführt, ebenso ist es möglich, dass eine Klebeverbindung zwischen den Komponenten ausgeführt ist. Alternativ zu einer separaten Ausgestaltung des Saugnapfes 30 ist es möglich aber nicht von der Erfindung umfasst, diesen direkt in die distale Anschlusskappe 16 zu integrieren, beispielsweise integral anzuformen oder im Rahmen eines 2K-Spritzgussverfahrens anzuspritzen. Die Abschlusskappe 16, die in einem gewissen Maße auch noch verformbar ist, kann aus dem Material des Prothesenliners 10 gefertigt sein und bei einer stoffschlüssigen Verbindung mit dem Saugnapf 30 ein zusammenhängendes System zwischen dem Prothesenliner 10 und dem Saugnapf 30 bilden.

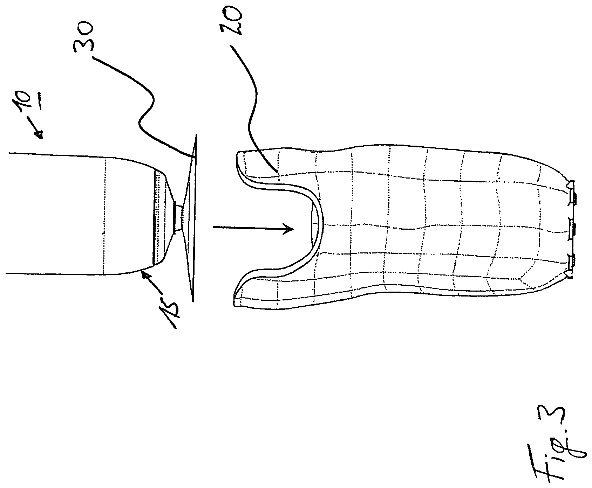

In der Figur 3 ist die Anwendung des Prothesenliners 10 mit dem an dessen Außenseite 15 angeordneten und daran befestigten Saugnapf 30 in Verbindung mit einem Prothesenschaft 20 dargestellt. Der Prothesenschaft 20 ist im Wesentlichen der äußeren Kontur des nicht dargestellten Stumpfes nachgebildet und im dargestellten Ausführungsbeispiel aus einem formstabilen luftdichten Werkstoff, beispielsweise einem faserverstärkten Kunststoff, ausgebildet. Der Anwender steigt mit dem Prothesenliner 10 und dem am distalen Ende 14 reversibel befestigten Saugnapf 30 durch die proximale Öffnung des Prothesenschaftes 20 in diesen hinein, dadurch wird durch die Aufbringung des Körpergewichtes in Distalrichtung auf den Saugnapf 30 eine Kraft aufgebracht. Auch wenn der Saugnapf 30, wie im dargestellten Beispiel ausgeführt, einen größeren Außendurchmesser als der Innendurchmesser des Prothesenschaftes 20 aufweist, kann der Saugnapf 30 in den Prothesenschaft 20 eingeführt werden, da der Saugnapf 30 aus einem elastischen Material ausgeführt ist, beispielsweise aus einem TPE, so dass es in diesem Fall zu einer Verformung des Saugnapfes 30 kommt. Durch das Körpergewicht wird der im Ausgangszustand mit einer konvexen, von dem Prothesenliner 10 weg gerichteten Kontur versehene Saugnapf 30 umgeklappt, so dass sich die Dichtlippe 31 mit der Ausbildung einer Dichtkante an der Innenseite des Prothesenschaftes 20 anlegt. Das Anlegen erfolgt an der gesamten Innenkontur des Prothesenschaftes 20, so dass zwischen dem Saugnapf 30 und dem Prothesenschaft ein Volumen eingeschlossen wird. Die innerhalb des Prothesenschaftes 20 eingeschlossene Luft wird an der Dichtlippe 31 vorbei beim Einführen des Prothesenliners 10 in Pfeilrichtung herausgedrückt, da der Prothesenschaft 20 luftdicht ausgebildet ist und ein geschlossenes distales Ende aufweist.

Zwischen dem Saugnapf 30 und der Prothesenschaftinnenseite wird daher ein Volumen aufgespannt, das durch eine Kraftaufbringung in Distalrichtung evakuiert werden kann. Durch den entstehenden Unterdruck z.B. in einer Schwungphase wird eine Haltekraft zwischen dem Saugnapf 30 und dem Prothesenschaft 20 erzeugt, so dass der Prothesenliner 10, der haftend an der Haut des Stumpfes liegt, über den Saugnapf 30, an dem der Prothesenliner 10 mechanische gekoppelt ist, an dem Prothesenschaft 20 gehalten wird.

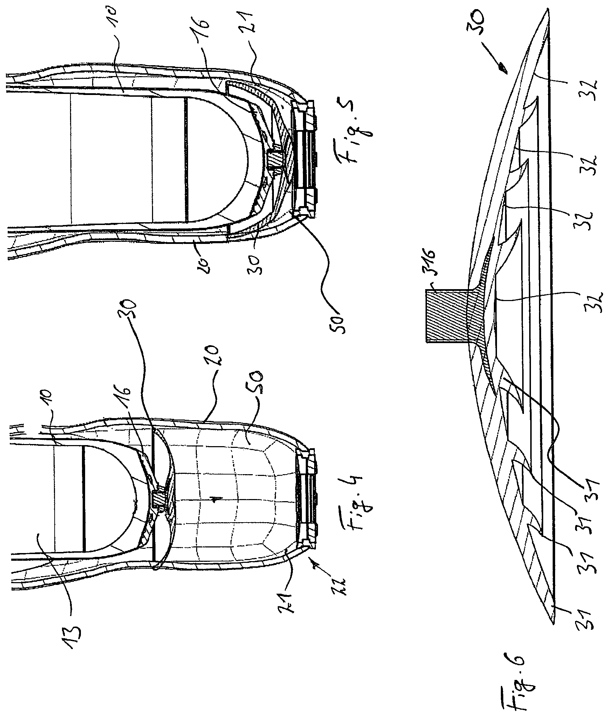

In der Figur 4 ist in der Schnittdarstellung ein teilweise eingeführter Prothesenliner 10 mit der distalen Abschlusskappe 16 und dem in dem Prothesenschaft 20 eingeführten Saugnapf 30 gezeigt. In der Schnittdarstellung ist auch der Aufnahmeraum 13 für den Stumpf des Prothesennutzers gezeigt. Durch die Druckbewegung in Distalrichtung wird der Saugnapf 30 zusammen mit dem Prothesenliner 10 in Richtung auf das distale Ende 22 des Prothesenschaftes 20 bewegt. Das zwischen dem Saugnapf 30 und dem distalen Ende 22 vorhandene, geschlossene Volumen 50 wird komprimiert, das innerhalb des Volumens 50 enthaltene Gas, in der Regel Luft, wird herausgedrückt und der Prothesenliner 10 kann weiter in Richtung auf das distale Ende 22 des Prothesenschaftes 20 verschoben werden, bis der Saugnapf 30 den am distalen Ende 22 befindliche Anlagebereich 21 erreicht.

In der Figur 5 ist die Endposition des Prothesenliners 10 und damit auch des Saugnapfes 30 innerhalb des Prothesenschaftes 20 dargestellt. Das Volumen 50 ist minimiert, die Dichtlippe des Saugnapfes 30 liegt an dem Anlagebereich 21 an, so dass bei Belastung in Proximalrichtung eine Volumenvergrößerung bewirkt wird, was dazu führt, dass der entstehende Unterdruck über die Fläche des Saugnapfes 30 eine Haltekraft ausübt, so dass der Prothesenliner 10 sicher in dem Prothesenschaft 20 gehalten wird.

Figur 6 zeigt in einer Schnittdarstellung den Saugnapf 30 in Einzeldarstellung. Neben den Befestigungsmitteln 316 in Gestalt eines eingearbeiteten Gewindes ist die konvexe Wölbung des unbelasteten, nicht eingeführten Saugnapfes 30 gut zu erkennen. An der Unterseite des Saugnapfes 30 sind mehrere umlaufende Dichtlippen 31 ausgebildet, die in Gestalt einer Lamellenstruktur angeordnet sind. Die Dichtlippen 31 sind konzentrisch zueinander angeordnet und erhöhen die Sicherheit, dass immer mindestens eine Dichtlippe 31 als Dichtkante in einem radial dichtenden Kontakt zu der Innenseite des Prothesenschaftes 20 bzw. an dem Anlagebereich 21 anliegt. Auf der Oberfläche der Unterseite des Saugnapfes 30 kann eine raue Struktur oder ein Gewebe auf der Saugnapfoberfläche 32 angeordnet oder ausgebildet sein, wodurch sichergestellt wird, dass nur die jeweils gewünschte Dichtlippe 31 in einen dichtenden Kontakt mit dem Prothesenschaft 20 tritt und die Wirkfläche des Vakuums über die Unterseite vollflächig verteilt wird. Die Unterseite des Saugnapfes 30 kann somit nicht in unbeabsichtigter Weise an einer anderen Stelle in dichtenden Kontakt mit dem Prothesenschaft 20 treten, so dass immer eine gleichmäßige Verteilung der Dichtkraft und ein minimales Niveau der Haltekraft erreicht wird.

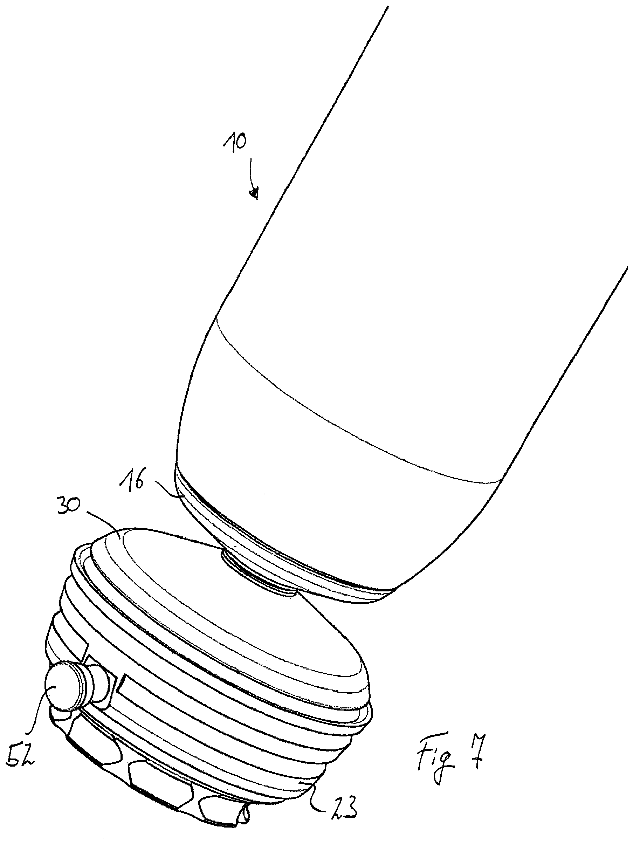

In der Figur 7 ist in einer perspektivischen Darstellung der Prothesenliner 10 mit der Anschlusskappe 16 und daran montierten Saugnapf 30 in einer noch nicht gefügten Position relativ zu der Aufnahmeschale 23 dargestellt. Die Aufnahmeschale 23 kann beispielsweise aus einem Leichtmetall oder einem Kunststoff hergestellt sein und bildet den distalen Abschluss des nicht weiter dargestellten Prothesenschaftes 20. Die Aufnahmeschale 23 kann am unteren, distalen Ende Anschlusseinrichtungen für zusätzliche Komponenten aufweisen, beispielsweise Gewinde oder dergleichen, um Oberteile eines Prothesengelenkes daran zu befestigen. Die Aufnahmeschale 23 ist mit einer Belüftungseinrichtung 52 in Gestalt eines Rückschlagventils ausgestattet, um einerseits eingeschlossene Luft aus dem Volumen zwischen dem Saugnapf 30 und der Aufnahmeschale 23 herausdrücken und bei einer gewünschten Trennung vom Prothesenschaft 20 das geschlossene Volumen wieder mit Luft befüllen zu können. Der Innendurchmesser der Aufnahmeschale 23 entspricht im Wesentlichen dem Außendurchmesser des Saugnapfes 30. Der Saugnapf 30 kann in seinem Außendurchmesser auch geringfügig größer oder kleiner ausgebildet sein. Ist der Außendurchmesser größer, wird bereits beim Aufsetzen auf den oberen Rand der Aufnahmeschale 23 das Volumen geschlossen, entspricht der Außendurchmesser dem Innendurchmesser der Aufnahmeschale 23, findet das Abschließen des Volumens erst im weiteren Verlauf der Einführbewegung statt, wenn der Saugnapf 30 in einem Bereich der Aufnahmeschale 23 angekommen ist, in dem sich die Aufnahmeschale 23 zum distalen Ende hin verjüngt. Die innere Kontur der Aufnahmeschale 23 ist becher- oder wannenartig ausgebildet, wobei die Seitenwände der Aufnahmeschale 23 am proximalen Rand im Wesentlichen senkrecht nach unten verlaufen, dann jedoch sich in Distalrichtung hin weiter verjüngen.

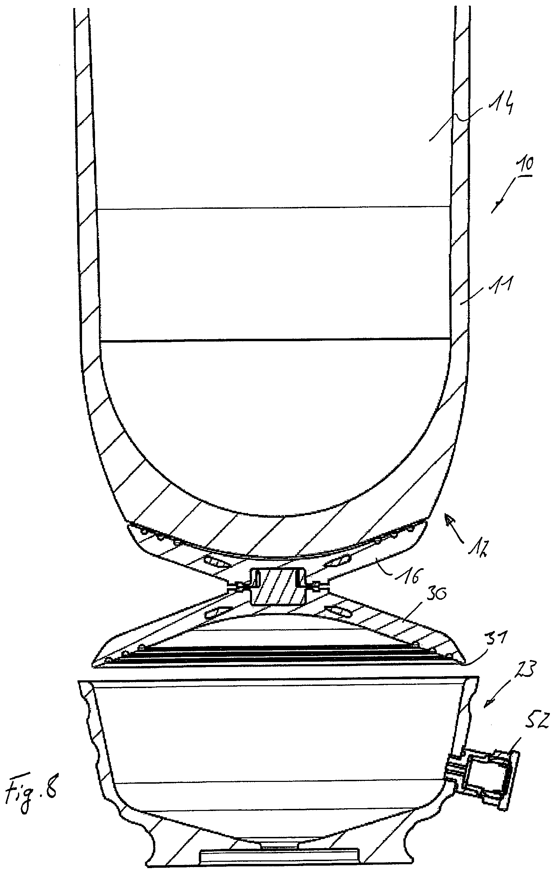

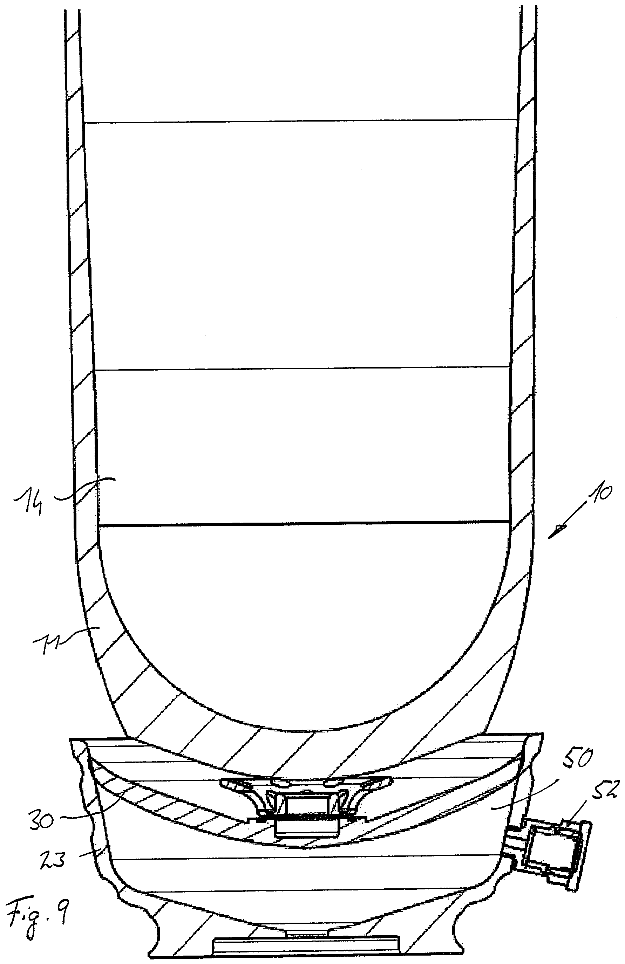

In der Figur 8 ist in einer Schnittdarstellung die Position des Saugnapfes 30 oberhalb der Aufnahmeschale 23 zu erkennen. Ebenso ist die schräge Wandung der Aufnahmeschale 23 in der in der Figur 8 dargestellten Ausführungsform zu erkennen, die konisch vom oberen, proximalen Rand sich verjüngend zum distalen Boden hin verläuft. Die Belüftungseinrichtung 52 stellt eine verschließbare Verbindung zur Umgebungsatmosphäre dar. Der Prothesenliner 10 mit dem elastischen Grundkörper 11, der beispielsweise aus Silikon oder einem Polymer ausgebildet sein kann, weist an seinem distalen Ende 12 die Abschlusskappe 16 auf, die an dem elastischen Grundkörper 11 angeformt oder auf eine andere Art und Weise daran befestigt sein kann. Der Saugnapf 30 mit der am äußeren Umfang umlaufenden Dichtlippe 31 weist eine im dargestellten Ausgangszustand vom Grundkörper 11 weg gekrümmte konvexe Kontur auf, die zusammen mit der Aufnahmeschale 23 ein Luftvolumen abschließen kann. In der Figur 9 ist der teilweise eingeführte Zustand des Saugnapfes 30 in die Aufnahmeschale 23 gezeigt. Der äußere Umfang mit der Dichtlippe 31 des Saugnapfes 30 liegt bereits dichtend an der inneren Oberfläche der Aufnahmeschale 23 an und schließt das Volumen 50 nach außen ab. Da die äußere Dichtlippe 31 bereits am oberen Rand der Aufnahmeschale 23 anlag, wird durch das weitere Einführen des Prothesenliners 10 über die zentrale Befestigungseinrichtung eine zentrale Kraft in Einführrichtung auf den elastischen Saugnapf 30 ausgeübt, was zu einem Umklappen bzw. Umbiegen des Saugnapfes 30 führt. In der Stellung gemäß Figur 9 weist der Saugnapf 30 eine konkave Form auf, das zwischen dem Saugnapf 30 und der Aufnahmeschale 23 eingeschlossene Volumen 50 steht in strömungstechnischer Verbindung zu der Belüftungseinrichtung 52, die gleichzeitig als Entlüftungseinrichtung ausgebildet sein kann.

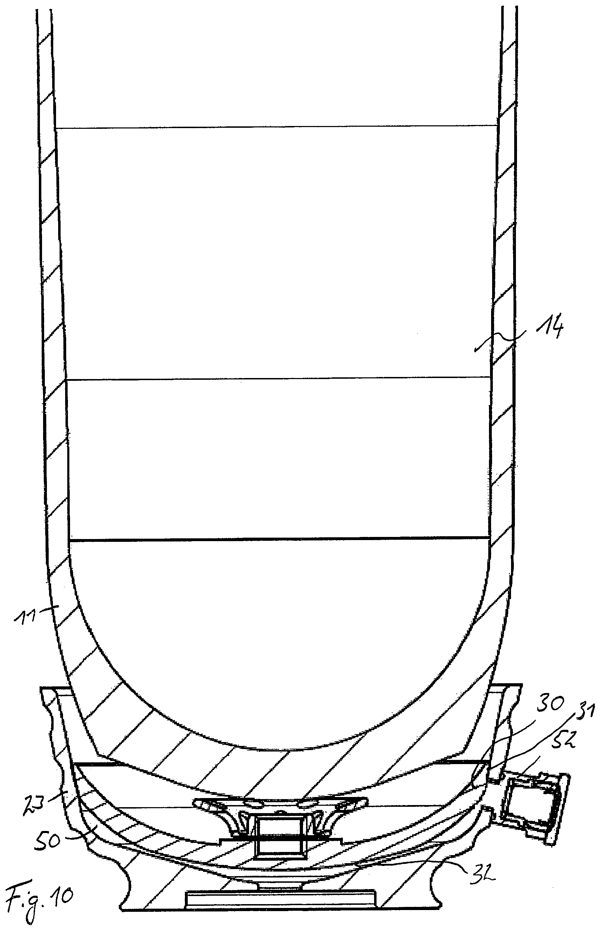

In der Figur 10 ist der vollständig eingeführte Zustand des Prothesenliners 10 und des Saugnapfes 30 in die Aufnahmeschale 23 dargestellt. Das eingeschlossene Volumen 50 ist minimal, der äußere Umfang und die Dichtlippe 31 liegen vollumfänglich und dichtend an der Innenseite der Aufnahmeschale 23 an, das Volumen 50 weist immerhin noch eine strömungstechnische Verbindung zu der Belüftungseinrichtung 52 auf. Sofern die Belüftungseinrichtung 52 verschlossen ist, kann aufgrund des luftdichten Abschlusses am Umfang des Saugnapfes 30 der Prothesenliner 10 nur unter sehr hohen Kräften entgegen der Haltekraft durch den Überdruck an der Außenseite des Saugnapfes von der Aufnahmeschale 23 getrennt werden. Die Oberfläche 32 des Saugnapfes 30 und/oder der Aufnahmeschale 23 können bereichsweise mit einer nicht haftenden oder luftleitenden bzw. aufgerauten Struktur versehen sein, um zu verhindern, dass der Saugnapf 30 auch an anderen Stellen ein Volumen einschließt, außer am gewünschten äußeren Umfang. Statt der separaten Aufnahmeschale 23 kann der Saugnapf 30 und der Prothesenliner 10 auch direkt in einen entsprechend ausgebildeten Prothesenschaft 20, der aus einem faserverstärkten Kunststoff oder einem ähnlichen Material hergestellt sein kann, eingebracht werden. Durch das Umklappen des Saugnapfes 30 von der ursprünglichen konvexen Form in eine konkave Form wird aufgrund der Rückstellkräfte neben der Haltekraft durch den Überdruck auch eine verstärkte Anpressung der Dichtlippe 31 an die Innenwand bzw. an den Anlagebereich 21 bewirkt.

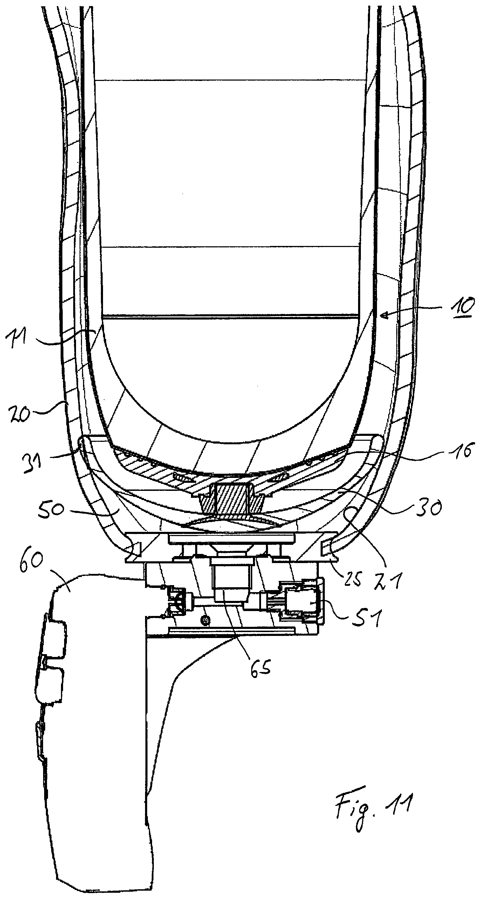

Eine Variante der Erfindung ist in der Figur 11 dargestellt, in der der Prothesenliner 10 in den Prothesenschaft 20 eingeführt dargestellt ist. In der Schnittdarstellung ist zu erkennen, dass der Saugnapf 30 von der konvexen Form in die konkave Form umgeklappt ist. Die Dichtlippe 31 liegt dichtend an dem Anlagebereich 21 auf der Innenseite des Prothesenschaftes 20 an. An dem distalen Ende ist eine Anschlussscheibe 25 angebracht, an der eine Vakuumpumpe 60 befestigt ist. Die Vakuumpumpe 60 steht über einen Kanal 65 in strömungstechnischer Verbindung mit dem Volumen 50. Zwischen der Anschlussscheibe 25 und dem Kanal 65 kann ein Rückschlagventil angeordnet sein, um ein Rückströmen herausgedrückter Luft zu vermeiden. Zum Lösen der Verbindung zwischen dem Prothesenschaft 20 und dem Saugnapf 30 ist es dann notwendig, eine Belüftungseinrichtung zu aktivieren. Ist kein Rückschlagventil in der Anschlussscheibe 25 angeordnet, wird das eingeschlossene Volumen 50 um den Kanal 65 erweitert. Die Luft kann dann aus dem Volumen 50 über die Evakuierungspumpe 60 abgezogen werden. Zum leichteren Einführen des Saugnapfes 30 ist eine Sperreinrichtung 51 in Gestalt eines Rückschlagventils vorgesehen, durch das Luft ausgestoßen werden kann, damit nicht die gesamte Luft aus dem Prothesenschaft 20 über die Vakuumpumpe 60 abtransportiert werden muss. Zum Belüften des Volumens 50 kann die Vakuumpumpe 60 auch in einem Druckbetrieb betreibbar sein, so dass Luft in das Volumen 50 eingeführt werden kann. Dazu ist es dann notwendig, das Entlüftungsventil 51 zu sperren, damit Luft in das eingeschlossene Volumen 50 hineingedrückt werden kann. Grundsätzlich ist es notwendig, dass eine Belüftungseinrichtung vorgesehen ist, die sich unterhalb der Dichtkante zwischen dem Saugnapf 30 und dem Prothesenschaft 20 befindet, um ein Belüften des evakuierten Volumens 50 und somit das Aussteigen aus der Prothese ermöglichen zu können. Im dargestellten Ausführungsbeispiel erfolgt der Abbau des Unterdruckes und das Fluten des eingeschlossenen Volumens 50 durch den distalen Zugang über das Ventil 51 und den Kanal 65.

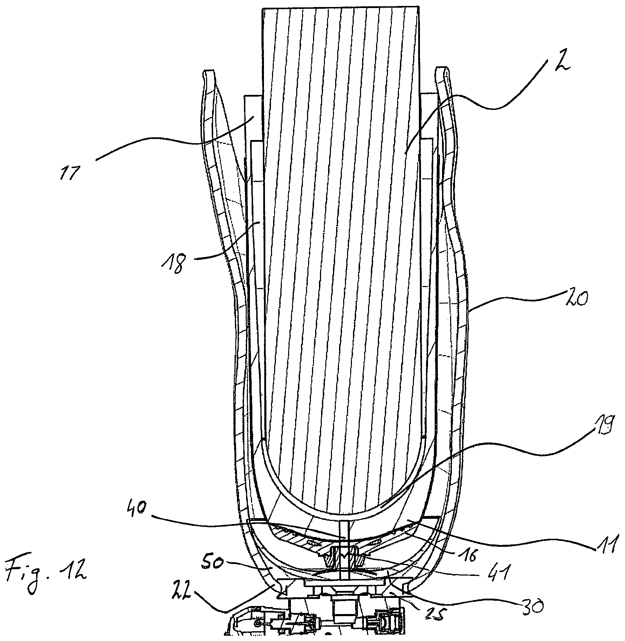

In der Figur 12 ist eine Variante der Figur 11, bei der die Anordnung unterhalb der Anschlussscheibe 25 identisch ausgebildet und nicht weiter dargestellt ist. Der Prothesenliner 10 weist an seinem proximalen Ende einen zum Stumpf 2 hin abdichtenden Abschnitt 17 auf, der als eine Dichtlippe fungiert. In distaler Richtung schließt sich an die Dichtlippe, die umlaufend ausgebildet ist, ein zurückgesetzter Bereich 18 an, der mit einem Stoff oder einer Gewebebeschichtung versehen sein kann oder auch nur einen Freiraum bildet, um den Aufbau eines Unterdruckes in diesem Zwischenbereich zu ermöglichen. Ein solcher Zwischenbereich kann auch durch das Anlegen eines Textils über den Stumpf 2 in diesem Bereich erfolgen. Dabei ist sicherzustellen, dass der abdichtende Abschnitt 17 dichtend an dem Stumpf 2 anliegt. Der Freiraum 19 zwischen dem Stumpf 2 und der Innenseite des Prothesenliners 10 steht in strömungstechnischer Verbindung durch einen Kanal 40 mit dem evakuierten Volumen 50 und über den Kanal 65 mit der Vakuumpumpe 60. Dadurch ist es möglich, dass über die Vakuumpumpe 60 zwischen dem Stumpf 2 und dem Prothesenliner 10 einen Unterdruck zu erzeugen. Das Volumen 50 kann über ein Rückschlagventil 41 von dem Volumen 19 zwischen dem Stumpf 2 und dem Liner 10 getrennt werden, so dass ein Rückströmen in das Volumen 19 zwischen dem Stumpf 2 und dem Liner 10 verhindert wird.

Eine Ausgestaltung gemäß der Figur 12 hat den Vorteil, dass der sogenannte Melkeffekt reduziert wird, der auftritt, wenn an dem distalen Ende 22 des Prothesenschaftes 20 eine Zugkraft aufgebracht wird. Durch das flächige Anliegen des Unterdruckes im gesamten Stumpfbereich wird die Krafteinleitung am Stumpf verbessert und Volumenschwankungen reduziert. Da der Unterdruck nicht zwischen dem Prothesenschaft 20 und dem Prothesenliner 10 anliegt, sondern zwischen dem Saugnapf 30 und dem Prothesenschaft 20, muss der Prothesenschaft 20 nicht die hohen Anforderungen an Passgenauigkeit und Dichtigkeit wie die herkömmlichen Unterdruckschäfte aufweisen.

Durch die Elastizität des Prothesenliners schmiegt sich dieser durch den wirksamen Unterdruck optimal an die Stumpfkontur an, ohne dabei Druckstellen oder Hämatome zu verursachen.

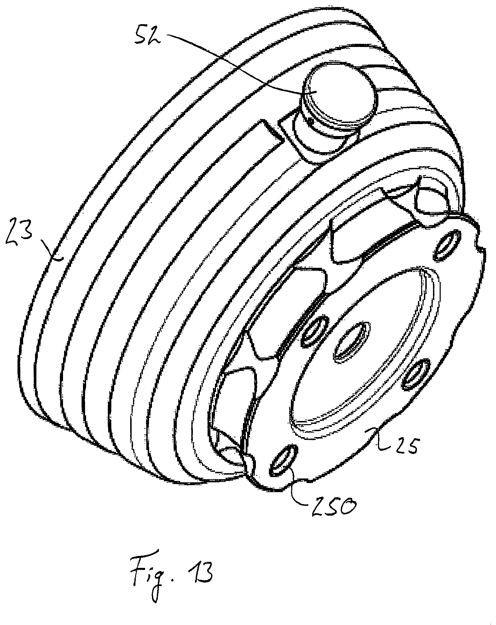

In der Figur 13 ist in einer perspektivischen Unteransicht die Aufnahmeschale 23 mit der Belüftungseinrichtung 52 und der Anschlussscheibe 25 dargestellt. In der Anschlussscheibe 25 können Gewinde 250 angeordnet sein, beispielsweise um eine Vakuumpumpe 60 oder andere Komponenten einer prothetischen Versorgung befestigen zu können.

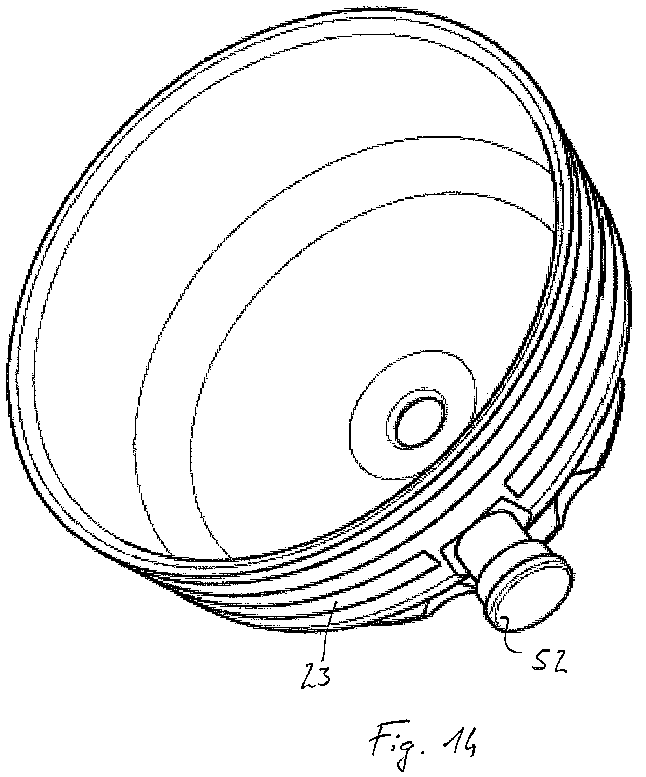

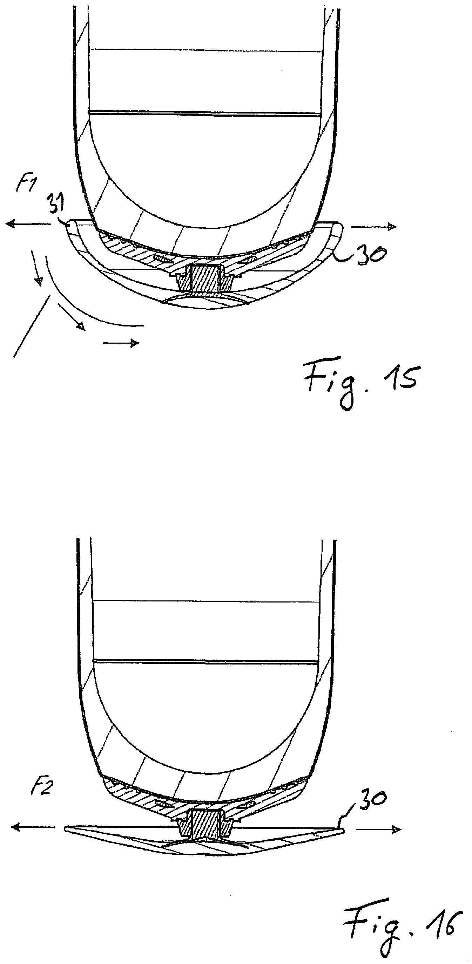

In der Figur 14 ist die Aufnahmeschale 23 in einer Schrägdraufsicht gezeigt, die konisch verlaufenden Seitenwände und die becherförmige Gestalt sowie die distale geschlossene Ausgestaltung mit der Möglichkeit, eine Durchgangsbohrung für eine Verbindung zu einer Vakuumpumpe 60 einzubringen, sind zu erkennen. In der Figur 15 sind schematisch die Mechanismen der Anlage des Saugnapfes 30 an die Aufnahmeschale 23 bzw. den Anlagebereich 21 des Prothesenschaftes 20 dargestellt. Fällt der Druck in dem evakuierten Volumen 50 zwischen dem Saugnapf 30 und dem Prothesenschaft 20 aus irgendeinem Grund ab, so führt dies zu einem erhöhten Spiel zwischen dem Saugnapf 30 und dem Prothesenschaft 20. Dies führt bei einer Belastung der Prothese in Distalrichtung, z.B. in einer Schwungphase, dazu, dass der Prothesenliner 10 leicht aus dem Prothesenschaft 20 herausbewegt wird, was zu einer Abrollbewegung der umgeklappten Dichtlippe 31 führt, wodurch diese stärker an die nicht dargestellte Schaftwand oder Wand der Aufnahmeschale 23 gepresst wird, wodurch sich die Dichtwirkung wiederum erhöht. Diese Funktion führt zu einer erhöhten Verliersicherung, die aufgrund der umgeklappten Kontur von der ursprünglich konvexen in die konkave im montierten Zustand herrührt. In der Figur 15 befindet sich der Saugnapf 30 noch in der vollständig eingeführten Position, die Dichtkraft F1 auf die Innenseite des Prothesenschaftes 20 ist relativ gering und durch die Rückstellkräfte aufgrund der elastischen Verformung beim Umklappen des Saugnapfes 30 zurückzuführen. Wird der Prothesenschaft 20 in distale Richtung relativ zu dem Saugnapf 30 verlagert, wird der Zentralbereich des Saugnapfes 30 angehoben und befindet sich auf einer Höhe mit der Dichtlippe 31, wodurch sich die Dichtkraft F2 wesentlich vergrößert. In der Figur 15 ist der Zustand gezeigt, wenn der Prothesenliner 10 spielfrei in dem Prothesenschaft 20 sitzt, die Figur 16 zeigt den Zustand, wenn zwischen dem Prothesenschaft 20 und dem Liner 10 ein Spiel vorhanden ist. Bei einem Wiederauftreten und einer distalen Verlagerung des Prothesenliners 10 in den Prothesenschaft 20 wird die in dem Volumen 50 eingeschlossene Luft herausgedrückt und die Haltekraft wieder erhöht.

Die dargestellte Erfindung hat gegenüber mechanischen Zapfenverriegelungen den Vorteil einer geringeren Aufbauhöhe. Das Prothesenschaftsystem kann somit auch dort eingesetzt werden, wo die Verwendung der sogenannten Shuttle-Lock-Systeme nicht möglich ist. Die Haltekraft zu den distalen Protheseneinrichtungen wird über den Prothesenschaft 20 zwischen dessen Innenseite und dem Befestigungssystem aus dem Saugnapf 30 und dem Prothesenliner 10 erzeugt. Die Halterung des Saugnapfes 30 an dem Prothesenliner 10 erfolgt über ein mechanisches Kopplungselement, ein Verkleben oder eine einstückige Ausgestaltung. Der sich an den Saugnapf 30 proximal anschließende Bereich des Prothesenliners 10 muss nicht mehr luftdicht an dem Prothesenschaft 20 anliegen. Dadurch, dass der Unterdruck nicht mehr unmittelbar zwischen dem Prothesenliner 10 und dem Prothesenschaft 20 anliegt, sondern die Zusatzeinrichtung in Gestalt des Saugnapfes 30 in Verbindung mit dem Prothesenschaft 20 die Haltekraft aufbringt, ist die Passgenauigkeit des Prothesenschaftes 20 und die Befestigung an dem Prothesenschaft 20 immer gewährleistet, unabhängig von Volumenschwankungen des Stumpfes 2. Wird der Unterdruck zwischen dem Prothesenliner 10 und der Schaftinnenwand aufgebaut, kann dies dazu führen, dass bei Volumenschwankungen des Stumpfes 2 der Dichtkontakt unterbrochen werden kann, was wiederum zu einem Lösen des Prothesenschaftes 20 von dem Prothesenliner 10 zur Folge hätte.

Aufgrund der Tatsache, dass nur an dem distalen Ende des Prothesenliners 10 der Saugnapf 30 angeordnet ist, wird der Prothesenliner 10 nicht notwendigerweise in unmittelbarem Kontakt mit der Innenseite des Prothesenschaftes 20 gebracht, wodurch der Prothesenliner 10 mechanisch weniger belastet wird. Gleiches gilt für das Weichgewebe am Stumpf 2 des Prothesennutzers, so dass die Bildung von Hämatomen vermieden wird.

## Patentansprüche

1. Prothesenliner (10) zur Anlage an einen Stumpf (2), mit einem elastischen Grundkörper (11), der eine proximale Öffnung (12) zum Einführen des Stumpfes (2) in einen Aufnahmeraum (13) und ein distales Ende (14) aufweist, wobei an der Außenseite (15) des Prothesenliners (10) zumindest ein Saugnapf (30) angeordnet ist und der Saugnapf (30) als separates Bauteil ausgebildet und an dem Prothesenliner (10) befestigt ist, **dadurch gekennzeichnet, dass** der Prothesenliner (10) dazu eingerichtet ist, an einem Prothesenschaft (20) über einen Unterdruck zwischen dem Saugnapf (20) und dem Prothesenschaft (20) festgelegt zu werden, wobei zwischen dem Prothesenliner (10) und dem Prothesenschaft (20) kein Unterdruck anliegt.

2. Prothesenliner nach Anspruch 1, **dadurch gekennzeichnet, dass** an dem distalen Ende (14) eine formstabile Abschlusskappe (16) angeordnet ist.

3. Prothesenliner nach Anspruch 1 oder 2, **dadurch gekennzeichnet, dass** der Saugnapf (30) reversibel an dem distalen Ende (14) befestigt ist.

4. Prothesenliner nach einem der voranstehenden Ansprüche, **dadurch gekennzeichnet, dass** der Saugnapf (30) zumindest eine umlaufende Dichtlippe (31) aufweist und innerhalb der umlaufenden Dichtlippe (31) an der Saugnapfoberfläche (32) eine aufgeraute Struktur, ein Textil und/oder eine nichthaftende Beschichtung vorgesehen ist.

5. Prothesenliner nach einem der voranstehenden Ansprüche, **dadurch gekennzeichnet, dass** der Saugnapf (30) auf der dem Prothesenliner (10) abgewandten Seite eine konvexe Form aufweist.

6. Prothesenliner nach Anspruch 5, **dadurch gekennzeichnet, dass** der Saugnapf (30) aus der im Ausgangszustand konvexen Form in eine konkave Form umklappbar ausgebildet ist.

7. Prothesenliner nach einem der voranstehenden Ansprüche, **dadurch gekennzeichnet, dass** eine strömungstechnische Verbindung (40) zwischen dem Aufnahmeraum (13) und dem Saugnapf (30) besteht.

8. Prothesenliner nach Anspruch 7, **dadurch gekennzeichnet, dass** in der strömungstechnischen Verbindung (40) eine Sperreinrichtung (41) angeordnet ist, die ein Rückströmen in den Aufnahmeraum (13) verhindert.

9. Prothesenliner nach einem der voranstehenden Ansprüche, **dadurch gekennzeichnet, dass** an einem proximalen Ende des Prothesenliners (10) ein zu dem Stumpf (2) dichtender Abschnitt (17) ausgebildet und distal dazu ein Bereich (18) angeordnet ist, der zurückgesetzt ausgebildet und/oder mit einer rauen Struktur, Kanälen, einem Textil (19), einem Schaumstoff oder für Luft durchlässigen Beschichtung versehen ist.

10. Prothesenschaftsystem mit einem Prothesenliner (10) nach einem der voranstehenden Ansprüche und einem Prothesenschaft (20), **dadurch gekennzeichnet, dass** der Prothesenschaft (20) einen zu dem Saugnapf (30) korrespondierenden Anlagebereich (21) aufweist und zwischen dem Saugnapf (30) und dem Anlagebereich (21) im gekoppelten Zustand ein geschlossenes Volumen (50) ausgebildet ist und zwischen dem Prothesenliner (10) und dem Prothesenschaft (20) kein Unterdruck anliegt.

11. Prothesenschaftsystem nach Anspruch 10, **dadurch gekennzeichnet, dass** das geschlossene Volumen (50) mit zumindest einer Sperreinrichtung (51) in strömungstechnischer Verbindung steht, die ein Ausströmen von Luft ermöglicht und ein Rückströmen verhindert.

12. Prothesenschaftsystem nach Anspruch 10 oder 11, **dadurch gekennzeichnet, dass** das geschlossene Volumen (50) mit einer Belüftungseinrichtung (52) zum Einleiten von Luft in strömungstechnischer Verbindung steht.

13. Prothesenschaftsystem nach einem der Ansprüche 10 bis 12, **dadurch gekennzeichnet, dass** eine Evakuierungspumpe (60) mit dem geschlossenen Volumen (50) in strömungstechnischer Verbindung steht.

14. Prothesenschaftsystem nach Anspruch 13, **dadurch gekennzeichnet, dass** die Evakuierungspumpe (60) in einem Druckbetrieb zur Befüllung des geschlossenen Volumens (50) betreibbar ist.

15. Prothesenschaftsystem nach einem der Ansprüche 10 bis 14, **dadurch gekennzeichnet, dass** an dem distalen Ende (22) des Prothesenschaftes (20) eine Aufnahmeschale (23) mit konkaver Wölbung angeordnet ist.

16. Prothesenschaftsystem nach einem der Ansprüche 10 bis 15, **dadurch gekennzeichnet, dass** zwischen dem Aufnahmeraum (13) und dem geschlossenen Volumen (50) eine strömungstechnische Verbindung (40) besteht.

17. Prothesenschaftsystem nach Anspruch 16, **dadurch gekennzeichnet, dass** in der strömungstechnischen Verbindung (40) eine Sperreinrichtung (41) angeordnet ist, die ein Rückströmen in den Aufnahmeraum (13) verhindert.

## Claims

1. Prosthesis liner (10) for placing on a stump (2), having an elastic main body (11) which has a proximal opening (12) for introducing the stump (2) into a receiving space (13), and a distal end (14), wherein at least one suction cup (30) is arranged on the outer side (15) of the prosthesis liner (10) and the suction cup (30) is configured as a separate component and fastened to the prosthesis liner (10), **characterized in that** the prosthesis liner (10) is designed to be fastened to a prosthesis socket (20) via a negative pressure between the suction cup (30) and the prosthesis socket (20), wherein between the prosthesis liner (10) and the prosthesis socket (20) no negative pressure is applied.

2. Prosthesis liner as claimed in claim 1, **characterized in that** a dimensionally stable end cap (16) is arranged at the distal end (14).

3. Prosthesis liner as claimed in claim 1 or 2, **characterized in that** the suction cup (30) is fastened reversibly to the distal end.

4. Prosthesis liner as claimed in one of the preceding claims, **characterized in that** the suction cup (30) has at least one circumferential sealing lip (31) and within the circumferential sealing lip (31), a roughened structure, a textile and/or a non-adhesive coating is provided on the suction cup surface (32).

5. Prosthesis liner as claimed in one of the preceding claims, **characterized in that** the suction cup (30) has a convex shape on the side facing away from the prosthesis liner (10).

6. Prosthesis liner as claimed in claim 5, **characterized in that** the suction cup (30) is configured to be turnable from the convex shape in the initial state into a concave shape.

7. Prosthesis liner as claimed in one of the preceding claims, **characterized in that** a fluidic connection (40) exists between the receiving space (13) and the suction cup (30).

8. Prosthesis liner as claimed in claim 7, **characterized in that** a blocking device (41) which prevents a return flow into the receiving space (13) is arranged in the fluidic connection (40).

9. Prosthesis liner as claimed in one of the preceding claims, **characterized in that** a section (17) that provides sealing with respect to the stump (2) is formed at a proximal end of the prosthesis liner (10), and a region (18) which is configured in a set-back manner and/or is provided with a rough structure, channels, a textile (19), a foam or air-permeable coating is arranged distally to said section (17).

10. Prosthesis socket system having a prosthesis liner (10) as claimed in one of the preceding claims and a prosthesis socket (20), **characterized in that** the prosthesis socket (20) has a contact region (21) corresponding to the suction cup (30) and a closed volume (50) is formed between the suction cup (30) and the contact region (21) in the coupled state and no negative pressure is applied between the prosthesis liner (10) and the prosthesis socket (20).

11. Prosthesis socket system as claimed in claim 10, **characterized in that** the closed volume (50) is fluidically connected to at least one blocking device (51), said fluidic connection allowing air to flow out and preventing a return flow.

12. Prosthesis socket system as claimed in claim 10 or 11, **characterized in that** the closed volume (50) is fludicially connected to an aeration device (52) for introducing air.

13. Prosthesis socket system as claimed in one of claims 10 to 12, **characterized in that** an evacuation pump (60) is fluidically connected to the closed volume (50).

14. Prosthesis socket system as claimed in claim 13, **characterized in that** the evacuation pump (60) is operable in pressure operation in order to fill the closed volume (50).

15. Prosthesis socket systems as claimed in one of claims 10 to 14, **characterized in that** a receiving bowl (23) with a concave curvature is arranged at the distal end (22) of the prosthesis socket (20).

16. Prosthesis socket system as claimed in one of claims 10 to 15, **characterized in that** a fluidic connection (40) exists between the receiving space (13) and the closed volume (50).

17. Prosthesis socket system as claimed in claim 16, **characterized in that** a blocking device (41) which prevents a return flow into the receiving space (13) is arranged in the fluidic connection (40).

## Revendications

1. Manchon prothétique (10) destiné à être appliqué sur un moignon (2), comprenant un corps de base élastique (11) qui présente une ouverture proximale (12) destinée à l'introduction du moignon (2) dans un espace de réception (13) et une extrémité distale (14), dans lequel au moins une ventouse (30) est disposée sur la face extérieure (15) du manchon prothétique (10) et la ventouse (30) est réalisée comme un élément séparé et fixée au manchon prothétique (10), **caractérisé en ce que** le manchon prothétique (10) est conçu pour être fixé à une emboîture de prothèse (20) par une dépression entre la ventouse (30) et l'emboîture de prothèse (20), aucune dépression n'étant appliquée entre le manchon prothétique (10) et l'emboîture de prothèse (20).

2. Manchon prothétique selon la revendication 1, **caractérisé en ce qu'**un capuchon d'extrémité (16) de forme stable est disposé à l'extrémité distale (14).

3. Manchon prothétique selon la revendication 1 ou 2, **caractérisé en ce que** la ventouse (30) est fixée de manière réversible à l'extrémité distale (14).

4. Manchon prothétique selon l'une des revendications précédentes, **caractérisé en ce que** la ventouse (30) présente au moins une lèvre d'étanchéité périphérique (31) et qu'une structure rugueuse, un textile et/ou un revêtement non adhérent est prévu(e) à l'intérieur de la lèvre d'étanchéité périphérique (31) sur la surface de ventouse (32).

5. Manchon prothétique selon l'une des revendications précédentes, **caractérisé en ce que** la ventouse (30) présente une forme convexe du côté éloigné du manchon prothétique (10).

6. Manchon prothétique selon la revendication 5, **caractérisé en ce que** la ventouse (30) est conçue de manière à être rabattable de la forme convexe à l'état initial en une forme concave.

7. Manchon prothétique selon l'une des revendications précédentes, **caractérisé en ce qu'**il existe une liaison fluidique (40) entre l'espace de réception (13) et la ventouse (30).

8. Manchon prothétique selon la revendication 7, **caractérisé en ce qu'**un dispositif d'arrêt (41), qui empêche un reflux dans l'espace de réception (13), est disposé dans la liaison fluidique (40).

9. Manchon prothétique selon l'une des revendications précédentes, **caractérisé en ce qu'**à une extrémité proximale du manchon prothétique (10) est réalisée une section (17) assurant l'étanchéité avec le moignon (2) et, distalement à celle-ci, est disposée une zone (18) qui est réalisée en retrait et/ou pourvue d'une structure rugueuse, de canaux, d'un textile (19), d'une mousse ou d'un revêtement perméable à l'air.

10. Système d'emboîture de prothèse comprenant un manchon prothétique (10) selon l'une des revendications précédentes et une emboîture de prothèse (20), **caractérisé en ce que** l'emboîture de prothèse (20) présente une zone d'application (21) correspondant à la ventouse (30) et un volume fermé (50) est formé entre la ventouse (30) et la zone d'application (21) à l'état accouplé, et aucune dépression n'est appliquée entre le manchon prothétique (10) et l'emboîture de prothèse (20).

11. Système d'emboîture de prothèse selon la revendication 10, **caractérisé en ce que** le volume fermé (50) est en liaison fluidique avec au moins un dispositif d'arrêt (51) qui permet un échappement d'air et empêche un reflux.

12. Système d'emboîture de prothèse selon la revendication 10 ou 11, **caractérisé en ce que** le volume fermé (50) est en liaison fluidique avec un dispositif de ventilation (52) servant à l'introduction d'air.

13. Système d'emboîture de prothèse selon l'une des revendications 10 à 12, **caractérisé en ce qu'**une pompe de mise sous vide (60) est en liaison fluidique avec le volume fermé (50).

14. Système d'emboîture de prothèse selon la revendication 13, **caractérisé en ce que** la pompe de mise sous vide (60) peut fonctionner dans un mode de refoulement pour remplir le volume fermé (50).

15. Système d'emboîture de prothèse selon l'une des revendications 10 à 14, **caractérisé en ce qu'**une coquille de réception (23) à courbure concave est disposée à l'extrémité distale (22) de l'emboîture de prothèse (20).

16. Système d'emboîture de prothèse selon l'une des revendications 10 à 15, **caractérisé en ce qu'**il existe une liaison fluidique (40) entre l'espace de réception (13) et le volume fermé (50).

17. Système d'emboîture de prothèse selon la revendication 16, **caractérisé en ce qu'**un dispositif d'arrêt (41), qui empêche un reflux dans l'espace de réception (13), est disposé dans la liaison fluidique (40).
